# EUROPÄISCHE PATENTSCHRIFT

(11) **EP 3 712 902 B1**
(45) Veröffentlichungstag und Bekanntmachung des Hinweises auf die Patenterteilung: **26.05.2021**
(21) Anmeldenummer: 19163502.8
(22) Anmeldetag: 18.03.2019
(51) Int. Cl.: G21K 1/10, A61B 6/06, A61B 6/00

(54) **FILTERSYSTEM ZUR LOKALEN ABSCHWÄCHUNG VON RÖNTGENSTRAHLUNG, RÖNTGENAPPARAT UND VERFAHREN ZUR LOKALEN VERÄNDERUNG DER INTENSITÄT VON RÖNTGENSTRAHLUNG**
FILTER SYSTEM FOR LOCAL ATTENUATION OF X-RAYS, X-RAY APPARATUS AND METHOD FOR LOCALLY MODIFYING THE INTENSITY OF X-RAY RADIATION
SYSTÈME FILTRANT DESTINÉ À L'AFFAIBLISSEMENT LOCAL DU RAYONNEMENT X, APPAREIL À RAYONS X ET PROCÉDÉ DE MODIFICATION LOCALE DE L'INTENSITÉ DU RAYONNEMENT X

(43) Veröffentlichungstag der Anmeldung: 23.09.2020
(73) Patentinhaber: Siemens Healthcare GmbH, 91052 Erlangen (DE)
(72) Erfinder: Baßler, Michael, 55127 Mainz (DE); Gransee, Rainer, 55257 Budenheim (DE); Rührig, Manfred, 91207 Lauf a.d. Pegnitz (DE); Schmidt, Oliver, 91052 Erlangen (DE); Vatheriparambil Mohandas, Vishnu, 5616 VD Eindhoven (NL)

(56) Entgegenhaltungen:
- JP-A- H02 257 942
- US-A- 4 856 042
- US-B1- 9 431 141

## Beschreibung

Die Erfindung betrifft ein Filtersystem zur lokalen Abschwächung von Röntgenstrahlung, einen Röntgenapparat und ein Verfahren zur lokalen Veränderung der Intensität von Röntgenstrahlung.

Bei Röntgenuntersuchungen von Patienten wird Röntgenstrahlung auf den zu untersuchenden Bereich des Körpers des Patienten gerichtet. Hierbei kann die Situation auftreten, dass der zu untersuchende Bereich ein lokal unterschiedliches Absorptionsverhalten für Röntgenstrahlen aufweist. Z.B. weisen Weichteilgewebe, Organe und Knochen jeweils ein anderes Absorptionsverhalten auf. Dies kann dazu führen, dass innerhalb einer Röntgenaufnahme die für die medizinische Untersuchung interessanten Bereiche nur undeutlich sichtbar sind.

Vor diesem Hintergrund und um eine Strahlungsdosis für den Patienten bei der Untersuchung möglichst gering zu halten werden üblicherweise Röntgenfilter zur lokalen Schwächung der Röntgenstrahlung eingesetzt. Beispielsweise beschreibt die DE 10 2012 206 953 B3 ein Röntgenfilter, das im Strahlengang eines Röntgenapparats angeordnet wird, mit einer zwischen einer Membran und einer Deckplatte angeordneten Röntgenstrahlung absorbierenden Flüssigkeit, wobei eine Schichtdicke der Flüssigkeit durch Stellelemente lokal veränderbar ist, um die Schwächung der Strahlung lokal einzustellen.

Die US 3 755 672 A beschreibt ein Röntgenfilter mit einer zwischen zwei Platten angeordneten Absorberflüssigkeit, wobei eine der Platten flexibel ist und mittels Servomotoren lokal ein Abstand zwischen den Platten veränderbar ist. Die US 9 966 159 B2 beschreibt ein Röntgenfilter, bei dem eine Absorberflüssigkeit durch elektrische Kräfte mit Hilfe einer Elektrodenanordnung verdrängt wird, um einen Strahlengang bereichsweise freizugeben. Die US 6 453 012 B2 beschreibt einen Röntgenapparat mit einem Filtersystem, das eine Filtereinrichtung mit mehreren Filterelementen in Form sich in Strahlungsrichtung erstreckender Rohre aufweist. Die Rohre werden zur lokalen Einstellung einer Abschwächung der Strahlung durch sogenanntes "Elektrowetting" von einem Ende her mit einer Absorberflüssigkeit befüllt. Die US 4 856 042 A beschreibt ferner ein Röntgenfilter mit einer zwischen einer oberen und einer unteren Platte gebildeten Kammer, in welcher sich radial von einer Öffnung der unteren Platte aus erstreckende Trennwände angeordnet sind. Durch die Öffnung kann der Kammer Alkohol zugeführt werden. Durch einen Stutzen kann der Kammer an einem radialen Randbereich Quecksilber zugeführt werden, so dass durch die Trennwände voneinander abgetrennte Teilräume in radialer Richtung zum Teil mit Alkohol und zum Teil mit Quecksilber gefüllt werden können. Die Druckschrift JP H02 257942 A beschreibt in Fig. 3 einen Strahlungsfilter mit mehreren parallelen Rohrkörpern, in welche Quecksilber eingefüllt ist, wobei zwischen zwei quecksilbergefüllten Bereichen ein Bereich mit einer für Strahlung durchlässigen Flüssigkeit gefüllt ist. Das Quecksilber bzw. der zwischen den Quecksilberbereichen gelegene Bereich mit einer für Strahlung durchlässigen Flüssigkeit kann durch Verändern eines Querschnitts am Ende des jeweiligen Rohrkörpers mittels eines Piezoelements innerhalb des Rohrkörpers verschoben werden.

Vor diesem Hintergrund besteht ein Bedarf, ein verbessertes Konzept für einen Röntgenfilter bereitzustellen, insbesondere ein Röntgenfilter, das einen einfachen Aufbau aufweist.

Diese Aufgabe wird durch die Merkmale der unabhängigen Ansprüche gelöst. Vorteilhafte Ausgestaltungen sind in den abhängigen Ansprüchen angegeben.

Gemäß einem ersten Aspekt der Erfindung ist ein Filtersystem zur lokalen Abschwächung von Röntgenstrahlung vorgesehen. Das Filtersystem umfasst eine Filtereinrichtung zur Anordnung im Strahlengang eines Röntgenapparats mit einer Kanalanordnung, welche eine Vielzahl sich in einer Ebene parallel zueinander erstreckender Kanalabschnitte aufweist, und eine Versorgungseinrichtung zum Bereitstellen eines 2-Phasen-Fluidstroms, welcher Tropfen aus einer Röntgenstrahlung absorbierenden Absorberflüssigkeit und eine für Röntgenstrahlung transparente Trägerflüssigkeit enthält.

Erfindungsgemäß ist vorgesehen, dass das Filtersystem eine Sortierstrecke mit einem mit der Versorgungseinrichtung verbundenen Eingang, mit einem mit der Kanalanordnung verbundenen ersten Ausgang, mit einem zweiten Ausgang und mit einer Ablenkeinrichtung zum Leiten einzelner Tropfen der Absorberflüssigkeit zu dem ersten Ausgang oder dem zweiten Ausgang aufweist. Der von der Versorgungseinrichtung bereitgestellte 2-Phasen-Fluidstrom wird der Sortierstrecke zugeführt, welche dazu eingerichtet ist, einzelne Tropfen, insbesondere Tropfen aus Absorberflüssigkeit aus dem Fluidstrom abzutrennen. Hierzu weist die Sortierstrecke eine Ablenkeinrichtung zum Aufbringen einer quer zur Strömungsrichtung gerichteten Kraft auf die Tropfen auf, so dass die Tropfen entweder einem mit der Kanalanordnung fluidisch leitend verbundenen ersten Ausgang oder einem nicht mit der Kanalanordnung, z.B. mit einem Reservoir verbundenen zweiten Ausgang zugeführt werden. Auf diese Weise können bestimmte Tropfenabfolgen effizient erzeugt werden. Beispielsweise kann die Ablenkeinrichtung eine erste Elektrode und eine dieser gegenüberliegend angeordnete zweite Elektrode aufweisen, um ein elektrisches Feld zum Ablenken der Tropfen in einem sich zwischen dem Eingang und den Ausgängen der Sortierstrecke erstreckenden Abscheidungsabschnitt zu erzeugen. Alternativ kann die Ablenkeinrichtung auch zur Erzeugung eines Druckpulses eingerichtet sein, um die einzelnen Tropfen abzulenken.

Mittels der Sortierstrecke können nahezu beliebige Tropfenabfolgen und damit Absorptionsmuster zur lokalen Schwächung der Röntgenstrahlung auf effiziente Weise erzeugt werden. Damit können die Bereiche, in denen die Röntgenstrahlung geschwächt werden soll, flexibel gewählt werden.

Eine der Erfindung zugrundeliegende Idee besteht darin, eine bestimmte Abfolge an Flüssigkeitstropfen eines Röntgenstrahlung absorbierenden Absorbermaterials und Flüssigkeitstropfen aus einem für Röntgenstrahlung transmittiven Trägermaterial, welches mit dem Absorbermaterial nicht mischbar ist, zu erzeugen, diese Abfolge an Tropfen in einem flächigen Kanalsystem zu positionieren und das Kanalsystem im Strahlengang eines Röntgenapparats anzuordnen. Dadurch werden die Röntgenstrahlen an den Stellen des Kanalsystems, an denen Tropfen aus Absorbermaterial angeordnet sind, ganz oder teilweise absorbiert bzw. abgeschwächt.

Zur Positionierung der Tropfen im Strahlengang ist eine Filtereinrichtung mit einer Kanalanordnung vorgesehen. Die Kanalanordnung weist eine flächige Erstreckung, die durch eine Vielzahl nebeneinander angeordneter, parallel verlaufender Kanalabschnitte definiert ist. Jedem Kanalabschnitt kann eine in Bezug auf die Längserstreckung des jeweiligen Kanalabschnitts vorbestimmte Tropfenabfolge zugeführt werden. Hierzu ist die Kanalanordnung an eine Versorgungseinrichtung angeschlossen.

Die Versorgungseinrichtung liefert einen zweiphasigen Fluidstrom, in welchem eine Phase durch Tropfen aus Absorberflüssigkeit und die andere Phase durch Tropfen aus Trägerflüssigkeit gebildet ist. Ein Vorteil der Erfindung liegt darin, dass die Filtereinrichtung, die zur Anordnung im Strahlengang vorgesehen ist, mit der Kanalanordnung konstruktiv sehr einfach aufgebaut ist und im Wesentlichen aus hydraulischen Leitungsstrukturen besteht.

Gemäß einer weiteren Ausführungsform des Filtersystems ist vorgesehen, dass die Versorgungseinrichtung ein erstes Reservoir mit der Absorberflüssigkeit, ein zweites Reservoir mit der Trägerflüssigkeit und einen Tropfengenerator zum Erzeugen des 2-Phasen-Fluidstroms aufweist, wobei der Tropfengenerator mit einem ersten Eingang mit dem ersten Reservoir, mit einem zweiten Eingang mit dem zweiten Reservoir und mit einem Ausgang mit dem Kanalanordnung verbunden ist. Gemäß dieser Ausführungsform werden die einzelnen Tropfen, aus denen sich der 2-Phasen-Fluidstrom zusammensetzt, durch einen Tropfengenerator erzeugt, welcher dazu eingerichtet ist, einen ersten Fluidstrom aus Absorberflüssigkeit und einen zweiten Fluidstrom aus Trägerflüssigkeit wechselnd abzuschnüren, um so eine gewünschte, optional periodische Tropfenabfolge zu erzeugen. Es ist denkbar, dass die Tropfenabfolge mit Tropfen verschiedener Länge erzeugt werden, um die hinsichtlich eines Absorptionsmusters gewünschte Abfolge an Tropfen bereitzustellen. Auch können periodische Tropfenabfolgen mit im wesentlichen gleichgroßen Tropfen und regelmäßigen Abständen erzeugt werden, die anschließend mit einer mit dem Ausgang des Tropfengenerators verbundenen Sortierstrecke gemäß dem gewünschten Absorptionsmuster sortiert werden, wie dies im Folgenden noch beschrieben wird.

Gemäß einer Ausführungsform kann der Tropfengenerator einen den ersten Eingang aufweisenden ersten Leitungsabschnitt und einen den zweiten Eingang aufweisenden zweiten Leitungsabschnitt aufweisen, wobei der erste und der zweite Leitungsabschnitt T-förmig ineinander einmünden. Demnach ist der Tropfengenerator als T-Stück ausgebildet, dem an einem ersten Eingang Absorberflüssigkeit und an einem zweiten Eingang Trägerflüssigkeit zugeführt wird. Dadurch kann ein besonders einfacher Aufbau erzielt werden. Optional kann der Tropfengenerator auch ein an den ersten Eingang angeschlossenes erstes Ventil und ein an den zweiten Eingang angeschlossenes zweites Ventil aufweisen, wobei die Ventile jeweils zwischen einem Öffnungs- und einem Schließzustand schaltbar sind, um den Fluiddurchfluss jeweils zu unterbrechen oder zuzulassen. Z.B. können die Ventile als Magnetventile ausgeführt sein. Durch die Ventile wird eine Einstellung der Tropfengröße erleichtert.

Gemäß einer weiteren Ausführungsform des Filtersystems ist vorgesehen, dass die Versorgungseinrichtung ein Reservoir mit einer Emulsion aus Tropfen der Absorberflüssigkeit und der Trägerflüssigkeit aufweist und das Reservoir mit dem Eingang der Sortierstrecke verbunden ist. Beispielsweise kann der Trägerflüssigkeit hierzu ein Stabilisator, z.B. PEG (Polyethylenglykol) oder Silikon-Öl, dem Sauerstoff entzogen wurde. Das Bereitstellen einer Emulsion in einem Reservoir vereinfacht weiter den Aufbau des Filtersystems.

Gemäß einer Ausführungsform des Filtersystems ist vorgesehen, dass die Kanalabschnitte der Kanalanordnung miteinander über Verbindungsabschnitte derart verbunden sind, dass diese einen durchgehenden Kanal bilden. Die Sortierstrecke kann mit deren erstem Ausgang insbesondere mit einem Eingang des Kanals verbunden sein. Demnach ist jeder Kanalabschnitt an einem Ende über einen beispielsweise U-förmigen Verbindungsabschnitt mit einem weiteren, benachbarten Kanalabschnitt verbunden. Somit wird ein mäanderförmiger, durchgehender Kanal mit parallelen Kanalabschnitten gebildet. Dies bietet den Vorteil, dass mit einer einzigen Sortierstrecke die gesamte Kanalanordnung bzw. jeder einzelne Kanalabschnitt der Kanalanordnung mit einem Tröpfchenmuster befüllt werden kann. Dies vereinfacht weiter den Aufbau des Filtersystems.

Alternativ können die Kanalabschnitte der Kanalanordnung jeweils durch einzelne Kanäle gebildet sein, die jeweils mit der Versorgungseinrichtung verbunden sind. In diesem Fall kann das Filtersystem eine der Anzahl der Kanalabschnitte entsprechende Anzahl an Sortierstrecken aufweisen, wobei jeder Kanal der Kanalanordnung mit jeweils einem ersten Ausgang jeweils einer Sortierstrecke verbunden ist. Demnach ist die Kanalanordnung durch eine Vielzahl einzelner, separater Kanäle bzw. Leitungen gebildet und jeder Kanal ist über jeweils eine diesem zugeordnete Sortierstrecke mit der Versorgungseinrichtung verbunden. Ein Vorteil dieser Gestaltung liegt darin, dass alle Kanalabschnitte der Kanalanordnung gleichzeitig mit Tropfenabfolgen befüllt werden können.

Gemäß einer weiteren Ausführungsform des Filtersystems ist vorgesehen, dass eine erste Gruppe der Kanalabschnitte der Kanalanordnung in einer ersten Ebene angeordnet ist und eine oder mehrere weitere Gruppen an Kanalabschnitten vorgesehen sind, die jeweils in zu der ersten Ebene parallelen Ebenen angeordnet sind. Demnach kann die Kanalanordnung mehrere Gruppen von sich parallel erstreckenden Kanalabschnitten aufweisen, wobei die Kanalabschnitte einer jeweiligen Gruppe sich in jeweils einer Ebene erstrecken, und wobei die Ebenen parallel zueinander sind. Beispielsweise können zwischen zwei und zehn Gruppen an Kanalabschnitten vorgesehen sein. Dies bietet den Vorteil, dass ein Grad der Abschwächung der Strahlung einstellbar ist, indem in den verschiedenen Ebenen Tröpfchen aus Absorbtionsflüssigkeit überlappend angeordnet werden.

Gemäß einer weiteren Ausführungsform weist die Kanalanordnung zumindest zwei Platten auf, die an deren Oberflächen aneinander anliegen, wobei an den Oberflächen jeweils Nuten ausgebildet sind, welche die Kanalabschnitte definieren. Insbesondere weist eine erste Platte an einer ersten Oberfläche erste Nuten auf, eine zweite Platte weist an einer zweiten Oberfläche zweite Nuten auf, welche korrespondierend zu den ersten Nuten verlaufen, wobei die erste Oberfläche der ersten Platte an der zweiten Oberfläche der zweiten Platte anliegt. Somit wird ein konstruktiv einfacher Aufbau der Kanalanordnung realisiert. Beispielsweise kann durch diesen Aufbau auch vorteilhaft eine Kanalanordnung mit Kanalabschnitten in mehreren Ebenen realisiert werden. Die Platten können beispielsweise aus einem Kunststoffmaterial, wie z.B. PMMA (Polymethylmethacrylat), Glas oder einem anderen für Röntgenstrahlung weitestgehend durchlässigen Material gebildet sein.

Gemäß einer Ausführungsform wird als Absorberflüssigkeit Quecksilber oder Galinstan verwendet. Die Trägerflüssigkeit kann insbesondere ein Öl, wie z.B. Silikon-Öl sein.

Zum Transport der Tropfen bzw. allgemein des 2-Phasen-Fluidstroms in die Kanalanordnung hinein und aus dieser heraus können beispielsweise hydraulische Druckerzeugungseinrichtungen, wie Pumpen, und optional Ventile vorgesehen sein. Beispielsweise kann das Reservoir mit der Emulsion oder das Reservoir mit Trägerflüssigkeit und das Reservoir mit Absorberflüssigkeit jeweils über eine Pumpe mit dem Eingang der Sortierstrecke bzw. des Tropfenerzeugers verbunden sein. Alternativ hierzu ist auch denkbar, die Kanalabschnitte mit einer Elektrodenanordnung zu versehen, wobei eine Gruppe von ersten Elektroden elektrisch isolierend an den Kanalabschnitten entlang diesen verteilt angeordnet ist und jeweils zumindest eine zweite Elektrode in jeweils einem Kanalabschnitt vorgesehen ist. Die zweite Elektrode kann geerdet sein. Die ersten Elektroden können in einer zeitlichen Abfolge nacheinander mit einer elektrischen Spannungsquelle verbunden werden. Wenn der Trägerflüssigkeit ein Elektrolyt zugesetzt wird oder die Absorberflüssigkeit elektrisch leitend ist, kann auf diese Weise das als "Elektrowetting" bekannte Prinzip zum Tropfentransport in den Kanalabschnitten eingesetzt werden. Alternativ ist auch denkbar, mittels einer Elektrodenanordnung ein durch entlang der Kanalabschnitte wanderndes elektrisches Feld zu erzeugen und die Tropfen aus Absorberflüssigkeit durch elektrostatische Kräfte zu bewegen. Allgemein kann eine an die Kanalabschnitte gekoppelte Transporteinrichtung zum Transportieren des 2-Phasen-Fluidstroms vorgesehen sein.

Gemäß einem Beispiel der Offenbarung ist ein Filtersystem zur Abschwächung von Röntgenstrahlung vorgesehen, mit einer Filtereinrichtung zur Anordnung im Strahlengang eines Röntgenapparats mit einer Kanalanordnung, welche eine Vielzahl sich in einer Ebene parallel zueinander erstreckender Kanäle aufweist, einem Reservoir mit einer Röntgenstrahlung absorbierenden Absorberflüssigkeit, wobei die Kanäle an entgegengesetzten Enden jeweils mit dem Reservoir verbunden sind, und mit einem Transportsystem zum Transportieren von Absorberflüssigkeit in die Kanäle.

Eine diesem Beispiel zugrundeliegende Idee besteht darin, einen einfachen Aufbau einer Filtereinrichtung durch sich parallel erstreckende einzelne Kanäle zu erzielen und diese Kanäle von entgegengesetzten Seiten her mit Flüssigkeitssäulen eines Absorberfluids, wie z.B. Galinstan oder Quecksilber, zu füllen. Somit kann bei einem konstruktiv sehr einfachen Aufbau eine lokale Abschwächung der Röntgenstrahlung durch die Flüssigkeitssäulen aus Absorberflüssigkeit erfolgen.

Gemäß diesem Beispiel ist die Kanalanordnung durch eine Vielzahl einzelner, separater Kanäle bzw. Leitungen gebildet. Jeder der Kanäle ist mit einem ersten Ende und einem entgegengesetzt zu diesem gelegenen zweiten Ende jeweils fluidisch leitend mit dem Reservoir verbunden. Ein Vorteil dieser Gestaltung liegt darin, dass alle Kanalabschnitte der Kanalanordnung gleichzeitig mit Absorberfluid von entgegengesetzten Seiten her befüllt werden können.

Gemäß einer Ausführungsform dieses Beispiels ist vorgesehen, dass die Absorberflüssigkeit eine elektrisch leitfähige Komponente aufweist, wobei das Transportsystem eine Elektrodenanordnung mit einer Vielzahl entlang der Kanäle angeordneter Elektroden und eine Schalteinrichtung aufweist, welche dazu eingerichtet ist, die Elektroden jeweils einzeln mit einer elektrischen Spannungsquelle zu verbinden. Demnach ist vorgesehen, die Absorberflüssigkeit über elektrische Kräfte zu transportieren, z.B. über elektrostatische Kräfte oder durch sogenanntes "Elektrowetting".

Das Phänomen des Elektrowetting beruht darauf, einen Kontaktwinkel zwischen einer Flüssigkeit, hier der Absorberflüssigkeit, und einer Oberfläche, hier der Oberfläche des Kanals, durch Anlegen eines elektrischen Potentials zu variieren. Beispielsweise kann elektrisch ein Oberflächenspannungsgradienten über die Länge eines Flüssigmetallpfropfens, der sich zwischen elektrolytischen Flüssigkeiten befinde, induziert werden, wodurch Marangoni-Kräfte entlang der Flüssig-Flüssig-Grenzfläche erzeugt werden und eine Bewegung des Pfropfens ausgelöst wird. Es kann aber auch die Flüssigkeit selbst elektrisch leitend sein. Zum Transport der Absorberflüssigkeit in den Kanälen kann die Elektrodenanordnung beispielsweise eine Vielzahl erster Elektroden aufweisen, die entlang der einzelnen Kanäle angeordnet sind und elektrisch von der Absorberflüssigkeit isoliert sind, z.B. indem die Kanäle aus elektrisch isolierendem Material gebildet sind und die Elektroden an einer Außenfläche der Kanäle befestigt sind. Die ersten Elektroden beaufschlagen die Kanalwände mit einem ersten elektrischen Potential. Eine zweite Elektrode ist im Inneren der Kanäle angeordnet und beaufschlagt die Absorberflüssigkeit mit einem zweiten elektrischen Potenzial. Die Schalteinrichtung verbindet die einzelnen ersten Elektroden nacheinander mit der Spannungsquelle, wodurch ein Transport des Absorberfluides entlang der Kanäle erzielt wird.

Zum Transport durch elektrostatische Kräfte können an jeden Kanal entlang des Längserstreckung verteilt eine Vielzahl einander gegenüberliegender erster und zweiter Elektroden angeordnet sein, so dass diese bei Beaufschlagung mit einer Spannung einen Kondensator bilden. Durch Verbinden der Elektroden nacheinander mit einer Spannungsquelle kann zum Transport der Absorberflüssigkeit ein sich entlang eines jeweiligen Kanals 123 bewegendes elektrisches Feld erzeugt werden.

Gemäß einer weiteren Ausführungsform dieses Beispiels ist vorgesehen, dass das Transportsystem durch ein Hydrauliksystem mit zumindest einer Pumpe gebildet ist, welche zwischen dem Reservoir und der Kanalanordnung angeordnet ist.

Gemäß einer weiteren Ausführungsform dieses Beispiels ist eine erste Gruppe von Kanälen in einer ersten Ebene angeordnet ist und eine oder mehrere weitere Gruppen von Kanälen sind jeweils in zu der ersten Ebene parallelen Ebenen angeordnet. Demnach kann die Kanalanordnung mehrere Gruppen von sich parallel erstreckenden Kanälen aufweisen, wobei die Kanäle einer jeweiligen Gruppe sich in jeweils einer Ebene erstrecken, und wobei die Ebenen parallel zueinander sind. Beispielsweise können zwischen zwei und zehn Gruppen an Kanälen vorgesehen sein. Dies bietet den Vorteil, dass ein Grad der Abschwächung der Strahlung einstellbar ist, indem in den verschiedenen Ebenen Flüssigkeitssäulen aus Absorbtionsflüssigkeit überlappend bzw. mit verschiedenen Längen angeordnet werden.

Gemäß einer weiteren Ausführungsform dieses Beispiels weist die Kanalanordnung zumindest zwei Platten auf, die an deren Oberflächen aneinander anliegen, wobei an den Oberflächen jeweils Nuten ausgebildet sind, welche die Kanalabschnitte definieren. Insbesondere weist eine erste Platte an einer ersten Oberfläche erste Nuten auf, eine zweite Platte weist an einer zweiten Oberfläche zweite Nuten auf, welche korrespondierend zu den ersten Nuten verlaufen, wobei die erste Oberfläche der ersten Platte an der zweiten Oberfläche der zweiten Platte anliegt. Somit wird ein konstruktiv einfacher Aufbau der Kanalanordnung realisiert. Beispielsweise kann durch diesen Aufbau auch vorteilhaft eine Kanalanordnung mit Kanalabschnitten in mehreren Ebenen realisiert werden. Die Platten können beispielsweise aus einem Kunststoffmaterial, wie z.B. PMMA (Polymethylmethacrylat), Glas oder einem anderen für Röntgenstrahlung weitestgehend durchlässigen Material gebildet sein.

Sowohl nach dem ersten Aspekt der Erfindung als auch nach dem zweiten Beispiel der Offenbarung können die Kanalabschnitte der Kanalanordnung der Filtereinrichtung insbesondere einen Durchmesser in einem Bereich zwischen 50 µm. und 5 mm aufweisen, bevorzugt zwischen 500 µm und 3 mm aufweisen. Die Querschnittsform der Kanalabschnitte kann kreisförmig sein. Bei einer von der Kreisform abweichenden Querschnittsform wird unter dem Durchmesser des Kanalabschnitts der Durchmesser eines Kreises verstanden, der dieselbe Querschnittsfläche aufweist wie der jeweilige Kanalabschnitt.

Gemäß einem zweiten Aspekt der Erfindung ist ein Röntgenapparat vorgesehen. Der Röntgenapparat umfasst eine Röntgenquelle zum Erzeugen und Abstrahlen von Röntgenstrahlung in einen Strahlengang, einen in dem Strahlengang angeordneten Röntgendetektor und ein Filtersystem gemäß dem ersten Aspekt der Erfindung, wobei die Filtereinrichtung in dem Strahlengang zwischen Röntgenquelle und Röntgendetektor angeordnet ist. Beispielsweise kann die Filtereinrichtung derart in dem Strahlengang angeordnet sein, dass sich die Kanalabschnitte quer zu dem Strahlengang erstrecken.

Gemäß einem dritten Aspekt der Erfindung ist ein Verfahren zur lokalen Veränderung der Intensität von Röntgenstrahlung vorgesehen. Das Verfahren kann insbesondere mit einem System gemäß dem ersten Aspekt der Erfindung bzw. einem Röntgenapparat gemäß dem zweiten Aspekt der Erfindung durchgeführt werden. Das Verfahren umfasst ein Erzeugen vorbestimmter Abfolgen von Tropfen aus einem 2-Phasen-Fluidstrom, welcher Tropfen aus einer Röntgenstrahlung absorbierenden Absorberflüssigkeit und eine für Röntgenstrahlung transparente Trägerflüssigkeit enthält, Zuführen der Tropfenabfolgen in Kanalabschnitte einer Kanalanordnung einer in einem Strahlengang zwischen einer Röntgenquelle und einem Röntgendetektor angeordneten Filtereinrichtung, wobei die Kanalanordnung eine Vielzahl sich in einer Ebene parallel zueinander erstreckender Kanalabschnitte aufweist. Die in Bezug auf das System und den Röntgenapparat offenbarten Vorteile gelten in analoger Weise auch für das Verfahren.

Gemäß einem weiteren Beispiel ist ein Filtersystem zur Abschwächung von Röntgenstrahlung vorgesehen, mit einer Filtereinrichtung zur Anordnung im Strahlengang eines Röntgenapparats, welche zwei zueinander parallel angeordnete, einen Zwischenraum definierende Platten aufweist, einer Versorgungseinrichtung zum Bereitstellen eines 2-Phasen-Fluidstroms, welcher Tropfen aus einer Röntgenstrahlung absorbierenden Absorberflüssigkeit und eine für Röntgenstrahlung transparente Trägerflüssigkeit enthält, wobei die Versorgungseinrichtung mit dem Zwischenraum verbunden ist. Optional weist das Filtersystem weiterhin eine Sortierstrecke mit einem mit der Versorgungseinrichtung verbundenen Eingang, einem mit dem Zwischenraum verbundenen ersten Ausgang, einem zweiten Ausgang und einer Ablenkeinrichtung zum Leiten einzelner Tropfen der Absorberflüssigkeit zu dem ersten Ausgang oder dem zweiten Ausgang. Die optionale Sortierstrecke und die Versorgungseinrichtung können wie oben beschrieben ausgebildet sein.

Gemäß diesem Beispiel sollen die Tropfen aus Absorberflüssigkeit mittels Elektrowetting in dem Zwischenraum zwischen den Platten verteilt werden. Zum Transport der Tropfen Absorberflüssigkeit in dem Zwischenraum kann eine Elektrodenanordnung beispielsweise eine Vielzahl erster Elektroden aufweisen, die in Form einer Matrix oder eine Arrays an einer der Platten angeordnet sind und elektrisch von der Absorberflüssigkeit isoliert sind, z.B. indem die Platten aus elektrisch isolierendem Material gebildet sind und die Elektroden an einer Au-βenfläche einer der Platten befestigt sind. Die ersten Elektroden beaufschlagen die Platte mit einem ersten elektrischen Potential. Eine oder mehrere zweite Elektroden sind in dem Zwischenraum angeordnet und beaufschlagen die Tropfen aus Absorberflüssigkeit mit einem zweiten elektrischen Potenzial. Eine Schalteinrichtung verbindet die einzelnen ersten Elektroden nacheinander mit einer elektrischen Spannungsquelle, wodurch ein Transport der Tropfen aus Absorberflüssigkeit innerhalb des Zwischenraums erzielt wird.

Eine diesem Beispiel zugrundeliegende Idee besteht darin, die Tropfen aus Absorberflüssigkeit pixelartig über eine Fläche zu verteilen, indem diese durch Elektrowetting innerhalb eines zwischen zwei Platten gebildeten Zwischenraums transportiert und angeordnet werden. Das Trägerfluid kann hierbei z.B. als elektrolytische Flüssigkeit ausgebildet sein.

Die Erfindung wird nachfolgend anhand von Ausführungsbeispielen mit Hilfe der Figuren näher erläutert. Es zeigen:
Figur 1 eine schematische Darstellung eines Röntgenapparats gemäß einem Ausführungsbeispiel der vorliegenden Erfindung;
Figur 2 eine schematische Darstellung in Form eines funktionalen Blockdiagramms eines Filtersystems gemäß einem Ausführungsbeispiel der vorliegenden Erfindung;
Figur 3 eine schematische Darstellung in Form eines funktionalen Blockdiagramms eines Filtersystems gemäß einem weiteren Ausführungsbeispiel der vorliegenden Erfindung;
Figur 4 eine schematische Schnittdarstellung eines Tropfenerzeugers des Filtersystems aus Figur 3;
Figur 5 eine schematische Darstellung einer Kanalanordnung einer Filtereinrichtung eines Filtersystems gemäß einem Ausführungsbeispiel der vorliegenden Erfindung in einer Draufsicht;
Figur 6 eine schematische Schnittdarstellung der Kanalanordnung aus Figur 5;
Figur 7 eine schematische Darstellung einer Filtereinrichtung eines Filtersystems gemäß einem weiteren Ausführungsbeispiel der vorliegenden Erfindung in einer Draufsicht;
Figur 8 eine schematische Schnittdarstellung der Kanalanordnung der Filtereinrichtung aus Figur 7;
Figur 9 eine abgebrochene Schnittansicht eines einer Kanalanordnung einer Filtereinrichtung eines Filtersystems gemäß einem weiteren Ausführungsbeispiel der vorliegenden Erfindung;
Figur 10 eine perspektivische Ansicht einer Platte der in Figur 9 dargestellten Kanalanordnung;
Figur 11 eine vergrößerte Detailansicht des in Figur 10 mit dem Buchstaben Y gekennzeichneten Bereichs;
Figur 12 eine schematische Ansicht eines Filtersystems gemäß einem weiteren Ausführungsbeispiel, welches nicht von der vorliegenden Erfindung umfasst ist;
Figur 13 eine Draufsicht auf eine Kanalanordnung des Filtersystems gemäß Figur 12; und
Figur 14 eine Schnittansicht der Kanalanordnung aus Figur 13.

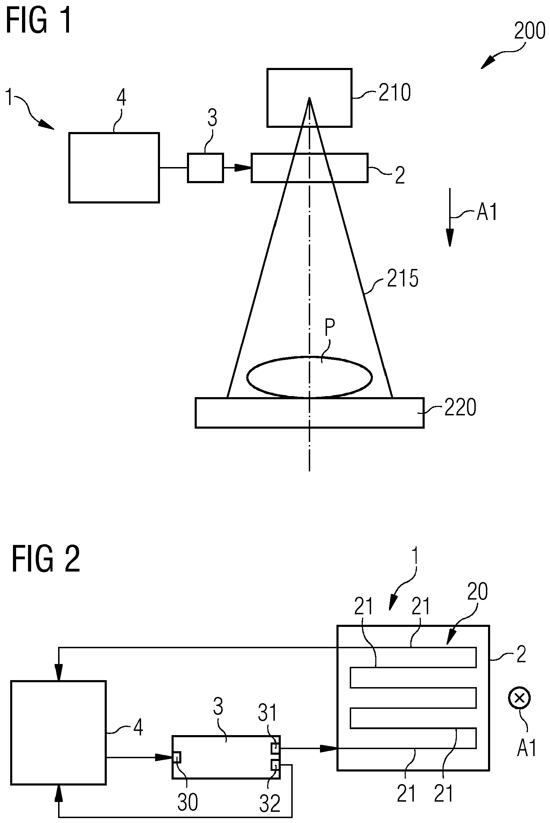

Figur 1 zeigt schematisch einen Röntgenapparat 200. Der Röntgenapparat 200 weist eine Röntgenquelle 210 zum Erzeugen und Abstrahlen von Röntgenstrahlung in einen Strahlengang 215, einen in dem Strahlengang 215 angeordneten Röntgendetektor 220 und ein Filtersystem 1 zur lokalen Abschwächung der Röntgenstrahlung auf. Wie in Figur 1 weiterhin schematisch dargestellt ist, weist das Filtersystem 1 eine Filtereinrichtung 2, eine Versorgungseinrichtung 4 und eine optionale Sortierstrecke 3 auf. Die Filtereinrichtung 2 in dem Strahlengang 215 zwischen der Röntgenquelle 210 und dem Röntgendetektor 220 angeordnet. Wie durch den Pfeil A1 in Figur 1 symbolisch dargestellt, durchdringt die von der Röntgenquelle 210 erzeugte Röntgenstrahlung zunächst die Filtereinrichtung 2, anschließend einen Patienten P und trifft dann auf den Röntgendetektor 220. Die Filtereinrichtung 2 dient dazu, die Röntgenstrahlung lokal abzuschwächen, um verschiedene Bereiche des Patienten P mit verschiedener Strahlungsintensität zu bestrahlen.

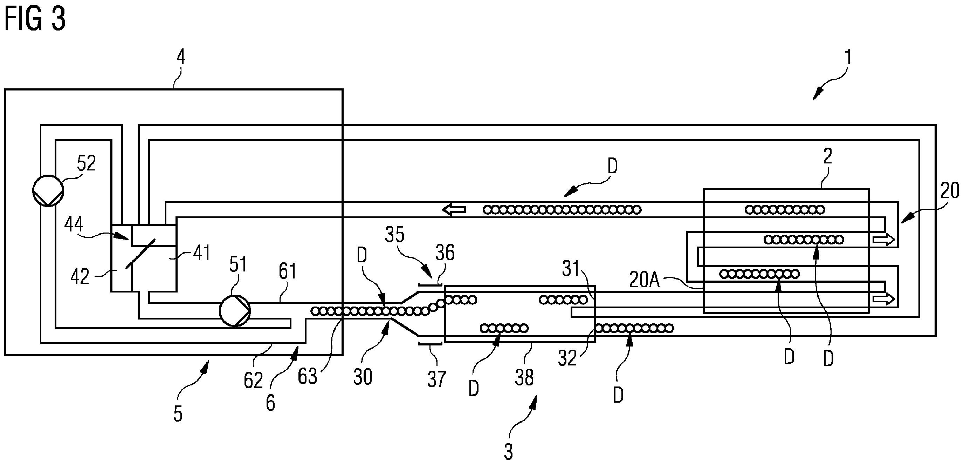

Figur 2 zeigt das Filtersystem 1 als schematische Blockdarstellung bzw. als vereinfachtes hydraulisches Flussbild. Figur 3 zeigt ein hydraulisches Flussbild des Filtersystems in einem größeren Detaillierungsgrad. Wie in Figur 2 dargestellt, umfasst das Filtersystem 1 eine Filtereinrichtung 2, eine Versorgungseinrichtung 4 und eine optionale Sortierstrecke 3.

Die Filtereinrichtung 2 weist eine flächige Kanalanordnung 20 auf, welche eine Vielzahl sich in einer Ebene parallel zueinander erstreckender Kanalabschnitte 21 umfasst. Wie in Figur 1 schematisch dargestellt und in Figur 1 durch das Richtungskreuz A1 dargestellt ist, kann die Filtereinrichtung 2 insbesondere derart in dem Strahlengang 215 angeordnet werden, dass sich die Kanalanordnung 20 quer zu dem Strahlengang 215 erstreckt.

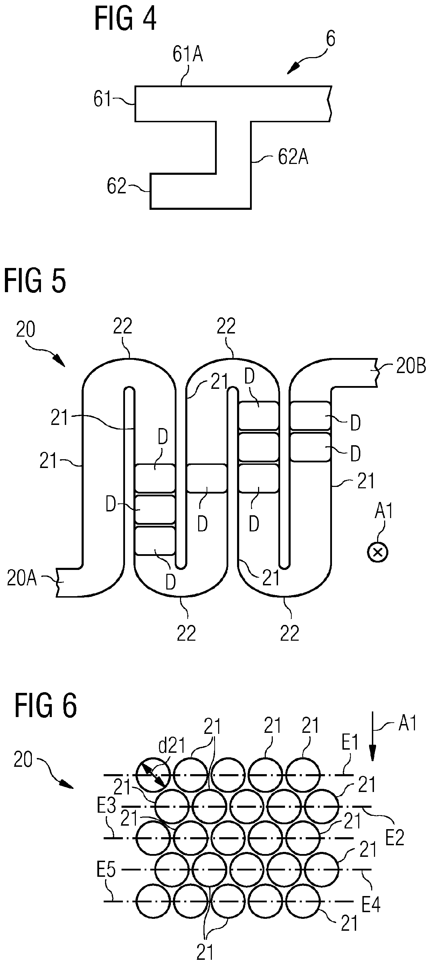

In den Figuren 2, 3 und 5 ist beispielhaft eine Kanalanordnung 20 in einer Draufsicht dargestellt. Wie insbesondere in Figur 5 erkennbar ist, können die parallelen Kanalabschnitte 21 der Kanalanordnung 20 an deren Enden miteinander über Verbindungsabschnitte 22, die z.B. U-förmig ausgebildet sind, derart verbunden sein, dass ein durchgehender Kanal gebildet wird. Die Kanalanordnung 20 kann demnach als ein durchgehender Kanal aus parallelen, vorzugsweise geraden Kanalabschnitten 21 und Verbindungsabschnitten 22 in Form eines flächigen Mäanders gebildet sein.

Figur 6 zeigt eine schematische Schnittansicht einer mäanderförmigen Kanalanordnung 20. Wie in Figur 6 beispielhaft dargestellt ist, können die Kanalabschnitte 21 jeweils einen kreisförmigen Querschnitt aufweisen. Ein Durchmesser d21 der Kanalabschnitte 21 kann allgemein zwischen 50 µm und 5 mm liegen. Wie in Figur 6 weiterhin beispielhaft dargestellt ist, ist eine erste Gruppe der Kanalabschnitte 21 der Kanalanordnung 20 in einer ersten Ebene E1 angeordnet, eine zweite Gruppe der Kanalabschnitte 21 der Kanalanordnung 20 ist in einer zweiten Ebene E2 angeordnet, eine dritte Gruppe der Kanalabschnitte 21 der Kanalanordnung 20 ist in einer dritten Ebene E3 angeordnet, eine vierte Gruppe der Kanalabschnitte 21 der Kanalanordnung 20 ist in einer vierten Ebene E4 angeordnet und eine fünfte Gruppe der Kanalabschnitte 21 der Kanalanordnung 20 ist in einer fünften Ebene E5 angeordnet. Die Ebenen E1-E5 erstrecken sich hierbei parallel zueinander. Allgemein können und eine oder mehrere weitere Gruppen an Kanalabschnitten 21 vorgesehen sein, die jeweils in zu der ersten Ebene E1 parallelen Ebenen E2-E5 angeordnet sind. Optional können die Kanalabschnitte 21 einer Ebene hierbei in einer Richtung quer zur Längserstreckung der Kanalabschnitte 21 versetzt zu den Kanalabschnitten 21 der benachbarten Ebenen angeordnet sein. Die Kanalabschnitte 21 jeweils einer Ebene E1-E5 sind über Verbindungsabschnitte 22 verbunden, wie dies in Figur 5 beispielhaft dargestellt wurde. Optional kann zusätzlich vorgesehen sein, dass jeweils ein Kanalabschnitt 21 einer Ebene über einen Verbindungsabschnitt 22 mit einem Kanalabschnitt 21 einer weiteren Ebene verbunden ist, so dass ein sich in allen Ebenen E1-E5 durchgehend erstreckender Kanal gebildet wird.

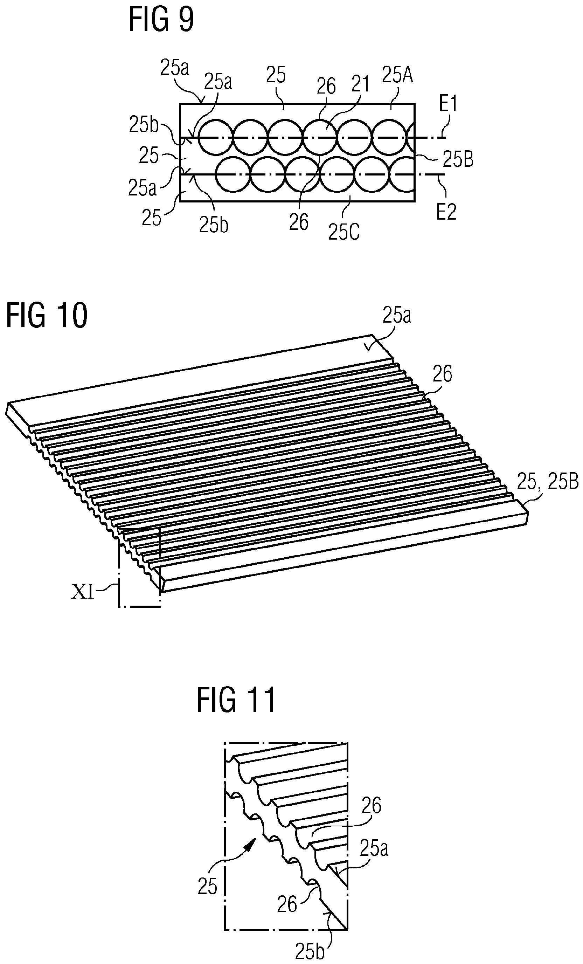

Die Kanalabschnitte 21 und gegebenenfalls die Verbindungsabschnitte 22 können als Kunststoffrohre ausgebildet werden, wie dies in den Figuren 5 und 6 beispielhaft und schematisch dargestellt ist. Alternativ ist auch denkbar, die Kanalanordnung 20 durch zumindest zwei Platten 25 auszubilden, die an deren Oberflächen 25a, 25b aneinander anliegen, wobei an den Oberflächen 25a, 25b jeweils Nuten 26 ausgebildet sind, welche die Kanalabschnitte 21 definieren, wie dies in Figur 9 beispielhaft dargestellt ist und nachfolgend noch im Detail erläutert wird.

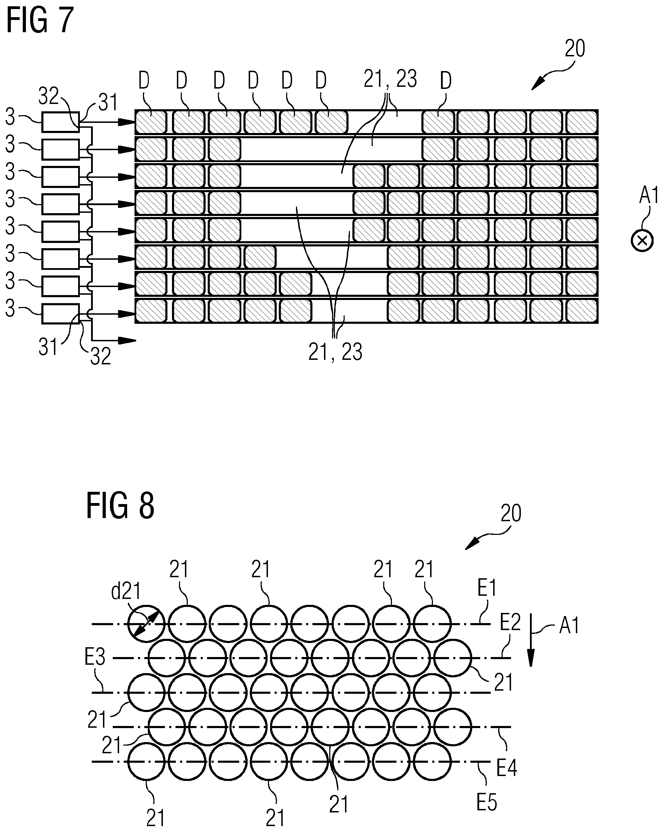

Wie in den Figuren 7 und 8 beispielhaft dargestellt ist, können die Kanalabschnitte 21 der Kanalanordnung 20 auch jeweils durch einzelne Kanäle 23 gebildet sein. Wie insbesondere in Figur 7 erkennbar ist, sind die Kanalabschnitte 20 durch parallel in einer Ebene verlaufende Kanalstrukturen, z.B. durch separate Rohre oder Leitungen gebildet, die untereinander nicht verbunden sind. Figur 8 zeigt eine Schnittansicht der in Figur 7 rein beispielhaft und schematisch dargestellten Kanalanordnung 20. Wie in Figur 8 beispielhaft dargestellt ist, können die Kanalabschnitte 21 jeweils einen kreisförmigen Querschnitt aufweisen. Ein Durchmesser d21 der Kanalabschnitte 21 kann allgemein zwischen 50 µm und 5 mm liegen. Wie in Figur 8 weiterhin beispielhaft dargestellt ist, ist eine erste Gruppe der Kanalabschnitte 21 der Kanalanordnung 20 in einer ersten Ebene E1 angeordnet, eine zweite Gruppe der Kanalabschnitte 21 der Kanalanordnung 20 ist in einer zweiten Ebene E2 angeordnet, eine dritte Gruppe der Kanalabschnitte 21 der Kanalanordnung 20 ist in einer dritten Ebene E3 angeordnet, eine vierte Gruppe der Kanalabschnitte 21 der Kanalanordnung 20 ist in einer vierten Ebene E4 angeordnet und eine fünfte Gruppe der Kanalabschnitte 21 der Kanalanordnung 20 ist in einer fünften Ebene E5 angeordnet. Die Ebenen E1-E5 erstrecken sich hierbei parallel zueinander. Allgemein können und eine oder mehrere weitere Gruppen an Kanalabschnitten 21 vorgesehen sein, die jeweils in zu der ersten Ebene E1 parallelen Ebenen E2-E5 angeordnet sind. Optional können die Kanalabschnitte 21 einer Ebene hierbei in einer Richtung quer zur Längserstreckung der Kanalabschnitte 21 versetzt zu den Kanalabschnitten 21 der benachbarten Ebenen angeordnet sein.

Figur 9 zeigt beispielhaft eine abgebrochene Schnittansicht einer Kanalanordnung 20, welche insgesamt drei Platten 25 aufweist. Die beispielhaft gezeigte Kanalanordnung 20 weist eine erste Gruppe von Kanalabschnitten 21 auf, in einer ersten Ebene E1 angeordnet sind, und eine zweite Gruppe von Kanalabschnitten 21, die in einer zweiten Ebene E2 angeordnet sind. Allgemein ist bei einer Anzahl von n Gruppen an Kanalabschnitten 21 in n Ebenen eine Anzahl von n+1 Platten 25 vorgesehen. Wie in Figur 9 beispielhaft dargestellt ist, sind an einer ersten Platte 25A an einer unteren Oberfläche 25b sich parallel erstreckende Nuten 26 ausgebildet. Die erste Platte 25A liegt mit ihrer unteren Oberfläche 25b an einer oberen Oberfläche 25a einer zweiten Platte 25B an, wobei an der oberen Oberfläche 25a der zweiten Platte 25B korrespondierend zu den Nuten 26 der ersten Platte 25B verlaufende Nuten 26 ausgebildet sind. Die Nuten 26 sind somit einander zugewandt und definieren gemeinsam den Querschnitt und die Längserstreckung der Kanalabschnitte 21. Bei der in Figur 9 beispielhaft dargestellten Kanalanordnung 20 weist die zweite Platte 25B an einer entgegengesetzt zu der oberen Oberfläche 25a orientierten unteren Oberfläche 25b weitere Nuten 26. Die zweite Platte 25B liegt mit ihrer unteren Oberfläche 25b an einer oberen Oberfläche 25a einer dritten Platte 25C an, wobei an der oberen Oberfläche 25a der dritten Platte 25B korrespondierend zu den Nuten 26 der ersten Platte 25B verlaufende Nuten 26 ausgebildet sind. Allgemein weist die Kanalanordnung 20 somit zumindest zwei Platten 25 auf, die an ihren Oberflächen 25a, 25b aneinander anliegen, wobei an den Oberflächen 25a, 25b jeweils Nuten 26 ausgebildet sind, welche die Kanalabschnitte 21 definieren.

Figur 10 zeigt beispielhaft eine Platte 25, welche z.B. die mittlere Platte 25B aus Figur 9 bilden kann. Wie in Figur 10 beispielhaft dargestellt ist, kann die Platte 25 in Bezug auf eine quer zur Längserstreckung der Nuten 26 verlaufende Querrichtung an entgegengesetzten Randabschnitten mit einer ebenen Oberfläche 25a ausgebildet sein. Dies erleichtert die Befestigung der Platten 25 aneinander, z.B. durch Verschweißen oder Verkleben. Die Platten 25 können insbesondere aus einem Kunststoffmaterial, wie z.B. PMMA (Polymethylmethacrylat), Glas oder einem anderen für Röntgenstrahlung weitestgehend durchlässigen Material gebildet sein. Figur 11 zeigt eine vergrößerte Detailansicht der in Figur 10 gezeigten Platte 25. Wie in Figur 11 insbesondere erkennbar ist, können die Nuten 26 an entgegengesetzten Oberflächen 25a, 25b einer jeweiligen Platte in einer quer zu ihrer Längserstreckung verlaufenden Richtung versetzt zueinander angeordnet sein.

Die Versorgungseinrichtung 4 dient zum Bereitstellen eines 2-Phasen-Fluidstroms, welcher Tropfen D aus einer Röntgenstrahlung absorbierenden Absorberflüssigkeit, wie z.B. Quecksilber oder Galinstan, und einer für Röntgenstrahlung transparenten Trägerflüssigkeit, wie z.B. Öl, insbesondere Silikon-Öl enthält. Figur 3 zeigt beispielhaft eine mögliche Gestaltung der Versorgungseinrichtung 4. Die in Figur 3 beispielhaft dargestellte Versorgungseinrichtung 4 weist ein erstes Reservoir 41, ein zweites Reservoir 42 und einen Tropfengenerator 6 sowie eine optionale Transporteinrichtung 5 mit einer ersten Pumpe 51 und einer zweiten Pumpe 52 auf.

In dem ersten Reservoir 41 ist die Absorberflüssigkeit aufgenommen. In dem zweiten Reservoir 42 ist die Trägerflüssigkeit aufgenommen. Das erste und das zweite Reservoir 41, 42 sind jeweils mit dem Tropfengenerator 6 fluidisch leitend verbunden. Insbesondere ist die erste Pumpe 51 in einem hydraulischen Pfad zwischen dem ersten Reservoir 41 und dem Tropfengenerator 6 angeordnet und die zweite Pumpe 52 ist in einem hydraulischen Pfad zwischen dem zweiten Reservoir 42 und dem Tropfengenerator 6 angeordnet, um die Flüssigkeiten aus den Reservoirs 41, 42 zu dem Tropfengenerator 6 zu transportieren. Optional können zwischen den Pumpen 51, 52 und dem Tropfengenerator 6 zusätzlich steuerbare Ventile (nicht dargestellt), wie z.B. Magnetventile angeordnet sein.

Wie in Figur 3 schematisch dargestellt ist, weist der Tropfengenerator 6 einen ersten Eingang 61, einen zweiten Eingang 62 und einen Ausgang 63 auf. Der erste Eingang 61 ist mit dem ersten Reservoir 41 und der zweite Eingang 62 mit dem zweiten Reservoir 42 verbunden. Der Ausgang 63 mit einem Eingang 30 der Sortierstrecke 3 verbunden. Figur 4 zeigt beispielhaft einen als T-Stück realisierten Tropfengenerator 6. Der Tropfengenerator 6 weist dabei einen den ersten Eingang 61 aufweisenden ersten Leitungsabschnitt 61A und einen den zweiten Eingang 62 aufweisenden zweiten Leitungsabschnitt 62A auf. Wie in Figur 4 schematisch dargestellt, mündet der erste Leitungsabschnitt 61A quer, vorzugsweise senkrecht in den zweiten Leitungsabschnitt 62A ein. Da die Absorberflüssigkeit und die Trägerflüssigkeit nicht mischbar sind, wird die Absorberflüssigkeit durch Einleiten der Trägerflüssigkeit von dem zweiten Leitungsabschnitt 62A in den ersten Leitungsabschnitt 61A abgeschnürt. Durch eine entsprechende Ansteuerung der Pumpen 51, 52 und/oder gegebenenfalls der Ventile kann in einfacher Weise eine periodische Abfolge an Tropfen aus Absorberflüssigkeit und aus Trägerflüssigkeit erzeugt werden. Damit bildet der Tropfengenerator 6 eine Einrichtung zum Erzeugen einer vorbestimmten Tropfenabfolge.

Alternativ zu einem Tropfengenerator 6 kann Versorgungseinrichtung 4 auch ein Reservoir 41 mit einer Emulsion aus Tropfen der Absorberflüssigkeit und der Trägerflüssigkeit aufweisen. Das Reservoir 41 kann beispielsweise analog zu dem ersten Reservoir 41 über die erste Pumpe 51 mit dem Eingang 30 der Sortierstrecke 3 verbunden sein. Zur Herstellung einer stabilen Emulsion kann der Trägerflüssigkeit ein Stabilisator zugesetzt sein, wie z.B. PEG (Polyethylenglykol) oder Silikon-Öl, dem Sauerstoff entzogen wurde.

Die optionale Sortierstrecke 3 ist in Figur 3 schematisch dargestellt. Die Sortierstrecke 3 weist einen Eingang 30, einen ersten Ausgang 31, einen zweiten Ausgang 32 und eine Ablenkeinrichtung 35 auf. Der Eingang 30 der Sortierstrecke 3 ist mit der Versorgungseinrichtung 4, z.B. mit dem Ausgang 63 des Tropfengenerators 6 wie in Figur 3 beispielhaft dargestellt oder mit direkt mit dem Reservoir 41 verbunden, wenn in dem Reservoir 41 eine Emulsion aus Tropfen der Absorberflüssigkeit und der Trägerflüssigkeit aufgenommen ist. Der erste Ausgang 31 ist mit einem Eingang 20A der Kanalanordnung 20 verbunden. Bei dem in Figur 3 beispielhaft dargestellten Filtersystem 1 ist die Kanalanordnung 20 durch einen durchgehenden mäanderförmigen Kanal gebildet, wie dies oben anhand der Figur 5 erläutert wurde. Es ist deshalb lediglich eine Sortierstrecke 3 vorgesehen, deren erster Ausgang 31 mit dem durchgehenden Kanal verbunden ist. der zweite Ausgang 32 kann insbesondere mit einem in Figur 3 lediglich symbolisch dargestellten Abscheider 44 verbunden sein, der dazu eingerichtet ist, die Absorberflüssigkeit von der Trägerflüssigkeit zu trennen, und der jeweils mit dem ersten und dem zweiten Reservoir 41, 42 verbunden ist. Ein Ausgang 20B der Kanalanordnung 20 kann ebenfalls mit dem Abscheider 44 verbunden sein, wie dies in Figur 3 beispielhaft gezeigt ist. Dadurch wird ein geschlossener Kreislauf realisiert, in welchem der 2-Phasen-Fluidstrom transportiert werden kann.

Bei der in Figur 7 beispielhaft dargestellten Kanalanordnung 20, bei welcher die Kanalabschnitte 21 durch einzelne Kanäle 23 realisiert sind, ist je Kanalabschnitt 21 bzw. je Kanal 23 jeweils eine Sortierstrecke 3 vorgesehen, deren erster Ausgang 31 mit einem Eingang des jeweiligen Kanals 23 verbunden ist. Die zweiten Ausgänge 32 der Sortierstrecken 3 können, analog zu der beispielhaften Darstellung in Figur 3, jeweils mit dem Abscheider 44 verbunden sein.

Die Ablenkeinrichtung 35 dient zum Leiten einzelner Tropfen D der Absorberflüssigkeit zu dem ersten Ausgang 31 oder dem zweiten Ausgang 32. Auf diese Weise kann jedem Kanalabschnitt 21 eine bestimmte Abfolge an Tropfen D aus Absorberflüssigkeit und Trägerflüssigkeit zugeführt werden. Wie in Figur 3 beispielhaft dargestellt ist, kann die Ablenkeinrichtung 35 eine erste Elektrode 36 und eine dieser gegenüberliegend angeordnete zweite Elektrode 37 aufweisen, um ein elektrisches Feld zum Ablenken der Tropfen in einem sich zwischen dem Eingang 30 und den Ausgängen 31, 32 der Sortierstrecke 3 erstreckenden Abscheidungsabschnitt 38 zu erzeugen. Wie in Figur 3 schematisch dargestellt ist, wird durch das mittels der Elektroden erzeugte elektrische Feld eine Richtungsänderung der Bewegung der Tropfen D aus Absorberflüssigkeit bewirkt, so dass diese entweder zum ersten Ausgang 31 und damit in die Kanalanordnung 20 oder zum zweiten Ausgang 32 und somit optional über den Abscheider 44 zurück ins erste Reservoir 41 geleitet werden. Die Ablenkeinrichtung 35 ist allgemein zum Aufbringen einer quer zur Strömungsrichtung gerichteten Kraft auf die Tropfen D eingerichtet. Damit bildet die Sortierstrecke eine weitere Einrichtung zum Erzeugen einer vorbestimmten Tropfenabfolge, welche alleine oder in Kombination mit dem Tropfengenerator 6 verwendet werden kann.

Zur lokalen Veränderung der Intensität der Röntgenstrahlung wird die Filtereinrichtung 2 wie in Figur 1 beispielhaft dargestellt im Strahlengang 215 des Röntgenapparats 200 angeordnet. Erfindungsgemäß mittels der Sortierstrecke 3 und mittels des Tropfengenerators 6 wird eine vorbestimmte Abfolge von Tropfen D aus dem durch die Versorgungseinrichtung 4 bereitgestellten 2-Phasen-Fluidstrom erzeugt. Die Tropfenabfolgen werden weiterhin in die Kanalabschnitte 21 der Kanalanordnung 20 transportiert bzw. diesen zugeführt, z.B. mittels des durch die Pumpen 51, 52 erzeugten hydraulischen Drucks oder mittels Elektrowetting durch eine an den Kanalabschnitten 21 vorgesehene Elektrodenanordnung. Alternativ ist auch denkbar die Tropfenabfolgen durch elektrostatische Kräfte, die durch an den Kanalabschnitten 21 vorgesehene Elektroden (nicht dargestellt) erzeugt werden, zu transportieren. Durch das Einführen von sortierten Abfolgen von Tropfen aus Absorberflüssigkeit und Tropfen aus Trägermaterial in die Kanalabschnitte 21 kann an diskreten Stellen, an denen die Tropfen aus Absorberflüssigkeit angeordnet sind, eine Schwächung der Strahlung erzielt werden. Figur 7 zeigt beispielhaft eine Anordnung von Tropfen D aus Absorberflüssigkeit. Die zwischen den Tropfenfolgen liegenden Bereiche sind durch Trägerflüssigkeit gefüllt, so dass in diesen Bereichen nur eine geringe oder keine Schwächung der Röngtenstrahlung erfolgt.

Die Sortiereinrichtung 3 kann auf effiziente Weise verschiedene Abfolgen an Tropfen bereitstellen. Die konstruktiv einfach aufgebaute Kanalstruktur 20 lässt sich vorteilhaft schnell befüllen und entleeren, z.B. durch Spülen mit Trägerflüssigkeit. Durch ihre flächige Erstreckung kann durch die Tropfen aus Absorbermaterial eine Art Pixelmuster zur ortsaufgelösten Schwächung der Strahlung erzeugt werden. Beim optionalen Vorsehen mehrerer Gruppen an Kanalabschnitten 21, die in verschiedenen Ebenen E1-E5 angeordnet sind, kann zudem der Grad der Schwächung für jedes Pixel individuelle eingestellt werden.

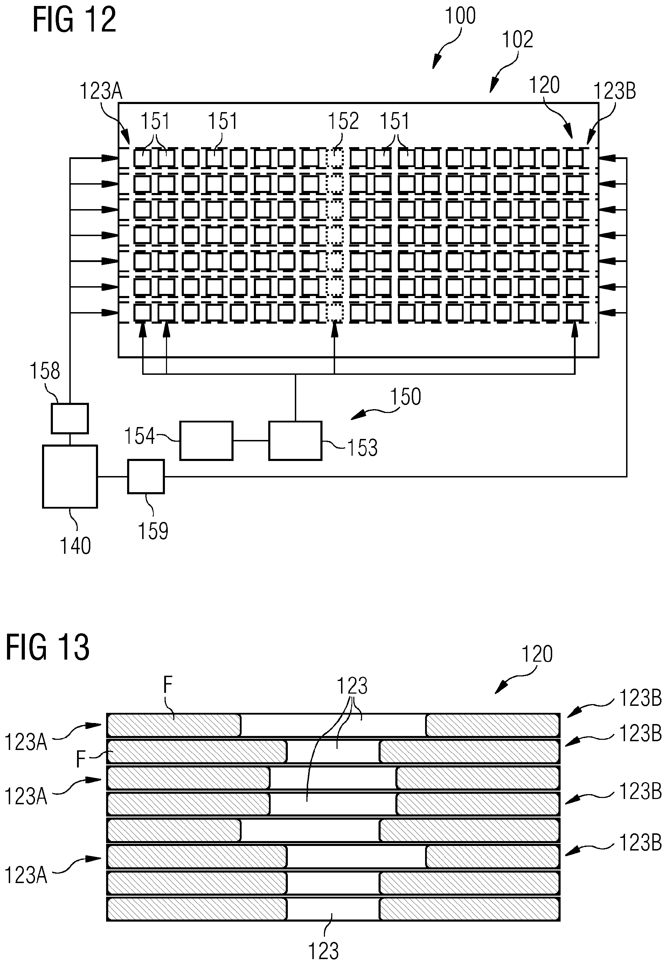

Figur 12 zeigt ein weiteres Filtersystem 100, das nicht Teil der Erfindung ist. Das Filtersystem 100 weist eine Filtereinrichtung 102 mit einer Kanalanordnung 120, ein Reservoir 140 mit einer Röntgenstrahlung absorbierenden Absorberflüssigkeit F und ein Transportsystem 150 auf.

Figur 13 zeigt eine Draufsicht auf das Kanalsystem 120. Wie insbesondere in Figur 13 erkennbar ist, sind die Kanäle 123 durch parallel in einer Ebene verlaufende Kanalstrukturen, z.B. durch separate Rohre oder Leitungen gebildet, die untereinander nicht verbunden sind. Jeder Kanal 123 erstreckt sich zwischen einem ersten Ende 123A und ein entgegengesetzt zu diesem gelegenen zweiten Ende 123B.

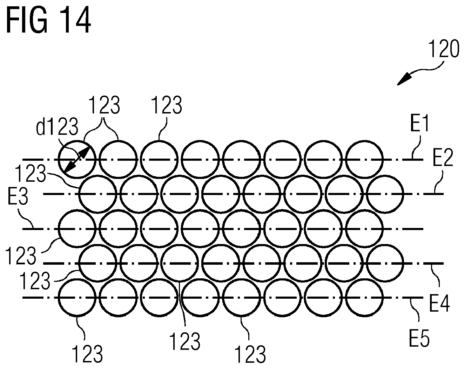

Figur 14 zeigt eine Schnittansicht der in Figur 13 rein beispielhaft und schematisch dargestellten Kanalanordnung 120. Wie in Figur 14 beispielhaft dargestellt ist, können die Kanäle 123 jeweils einen kreisförmigen Querschnitt aufweisen. Ein Durchmesser d123 der Kanäle 123 kann allgemein zwischen 50 µm und 5 mm liegen. Wie in Figur 14 weiterhin beispielhaft dargestellt ist, ist eine erste Gruppe der Kanäle 123 der Kanalanordnung 120 in einer ersten Ebene E1 angeordnet, eine zweite Gruppe der Kanäle 123 der Kanalanordnung 120 ist in einer zweiten Ebene E2 angeordnet, eine dritte Gruppe der Kanäle 123 der Kanalanordnung 120 ist in einer dritten Ebene E3 angeordnet, eine vierte Gruppe der Kanäle 123 der Kanalanordnung 120 ist in einer vierten Ebene E4 angeordnet und eine fünfte Gruppe der Kanäle 123 der Kanalanordnung 120 ist in einer fünften Ebene E5 angeordnet. Die Ebenen E1-E5 erstrecken sich hierbei parallel zueinander. Allgemein können und eine oder mehrere weitere Gruppen an Kanälen 123 vorgesehen sein, die jeweils in zu der ersten Ebene E1 parallelen Ebenen E2-E5 angeordnet sind. Optional können die Kanäle 123 einer Ebene hierbei in einer Richtung quer zur Längserstreckung der Kanäle 123 versetzt zu den Kanälen 123 der benachbarten Ebenen angeordnet sein, wie dies in Figur 14 beispielhaft dargestellt ist. Die einzelnen Kanäle 123 können beispielsweise wie in Figur 9 dargestellt und oben erläutert durch Platten 25 mit daran ausgebildeten Nuten 26 realisiert sein.

Das Reservoir 140 ist in Figur 12 lediglich symbolisch als Block dargestellt und enthält eine für Röntgenstrahlung absorbierende Flüssigkeit bzw. eine Absorberflüssigkeit, wie z.B. Galinstan oder Quecksilber. Der Absorberflüssigkeit kann beispielsweise ein Elektrolyt zugesetzt sein. Das Reservoir 140 ist mit der Kanalanordnung 120 fluidisch leitend verbunden, z.B. über ein Leitungssystem, wobei jeder Kanal 123 sowohl mit dem ersten Ende 123A als auch mit dem zweiten Ende 123B mit dem Reservoir 140 verbunden ist.

Wie in Figur 12 schematisch dargestellt ist, kann das Transportsystem 150 durch eine Elektrodenanordnung gebildet sein. Bei dem in Figur 12 beispielhaft dargestellten Filtersystem 100 weist die Elektrodenanordnung eine Vielzahl erster Elektroden 151 und je Kanal 123 zumindest eine zweite Elektrode 152 auf. Je Kanal 123 ist eine Vielzahl erster Elektroden 151 entlang des Kanals 123 verteilt angeordnet, z.B. an einer Au-βenseite der Kanäle 123 befestigt, um die Kanalwände mit einem ersten elektrischen Potential zu beaufschlagen. Die ersten Elektroden 151 sind elektrisch von der Absorberflüssigkeit isoliert, z.B. indem die Kanäle 123 aus elektrisch isolierendem Material gebildet sind. Je Kanal 123 ist zumindest eine zweite Elektrode 152 im Inneren des jeweiligen Kanals 123 angeordnet und beaufschlagt die Absorberflüssigkeit F mit einem zweiten elektrischen Potential.

Eine Schalteinrichtung 153 ist elektrisch mit den ersten Elektroden 151 verbunden bzw. dazu eingerichtet jede einzelne der ersten Elektroden 151 mit einer elektrischen Spannungsquelle 154 zu verbinden und optional das elektrische Potential der ersten Elektroden 151 zu steuern. Optional ist die Schalteinrichtung 153 auch mit den zweiten Elektroden 152 verbunden, um deren elektrische Potential zu steuern. Alternativ können die zweiten Elektroden 152 auch an ein Erdungspotential gekoppelt sein. Die Schalteinrichtung 153 ist dazu eingerichtet, die einzelnen ersten Elektroden 153 nacheinander mit der Spannungsquelle 154 zu verbinden, wodurch ein Transport des Absorberfluides entlang der Kanäle erzielt wird.

Alternativ kann das Transportsystem 150 auch durch ein Hydrauliksystem realisiert sein. Das Hydrauliksystem umfasst zumindest eine Pumpe, die zwischen dem Reservoir 123 und der Kanalanordnung 120 angeordnet ist, um die Kanäle 123 von zwei Seiten her mit einem bestimmten Füllstand an Absorberflüssigkeit F zu füllen. In Figur 12 ist beispielhaft ein Hydrauliksystem mit einer ersten Pumpe 158, die zwischen dem Reservoir 140 und den ersten Enden 123A der Kanäle 123 angeordnet ist, und einer zweiten Pumpe 159, die zwischen dem Reservoir 140 und den zweiten Enden 123B der Kanäle 123 angeordnet ist, dargestellt. Es ist auch denkbar, für jeden der Kanäle 123 eine oder zwei eigene Pumpen bereitzustellen. Alternativ oder zusätzlich können Ventile vorgesehen sein, um den Füllstand der Kanäle mit Absorberflüssigkeit zu steuern.

Obwohl die Erfindung im Detail durch die Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Filtersystem (1) zur lokalen Abschwächung von Röntgenstrahlung, mit:
einer Filtereinrichtung (2) zur Anordnung im Strahlengang (215) eines Röntgenapparats (200) mit einer Kanalanordnung (20), welche eine Vielzahl sich in einer Ebene parallel zueinander erstreckender Kanalabschnitte (21) aufweist;
einer Versorgungseinrichtung (4) zum Bereitstellen eines 2-Phasen-Fluidstroms, welcher Tropfen (D) aus einer Röntgenstrahlung absorbierenden Absorberflüssigkeit und eine für Röntgenstrahlung transparente Trägerflüssigkeit enthält; und
einer Sortierstrecke (3) mit einem mit der Versorgungseinrichtung (4) verbundenen Eingang (30), einem mit der Kanalanordnung (20) verbundenen ersten Ausgang (31), einem zweiten Ausgang (32) und einer Ablenkeinrichtung (35) zum Leiten einzelner Tropfen (D) der Absorberflüssigkeit zu dem ersten Ausgang (31) oder dem zweiten Ausgang (32).

2. Filtersystem (1) nach Anspruch 1, wobei die Kanalabschnitte (21) der Kanalanordnung (20) miteinander über Verbindungsabschnitte (22) derart verbunden sind, dass diese einen durchgehenden Kanal bilden.

3. Filtersystem (1) nach Anspruch 1, wobei die Kanalabschnitte (21) der Kanalanordnung (20) jeweils durch einzelne Kanäle (23) gebildet sind, die jeweils mit der Versorgungseinrichtung verbunden sind.

4. Filtersystem (1) nach einem der voranstehenden Ansprüche, wobei eine erste Gruppe der Kanalabschnitte (21) der Kanalanordnung (20) in einer ersten Ebene (E1) angeordnet ist und eine oder mehrere weitere Gruppen an Kanalabschnitten (21) vorgesehen sind, die jeweils in zu der ersten Ebene (E1) parallelen Ebenen (E2-E5) angeordnet sind.

5. Filtersystem (1) nach einem der voranstehenden Ansprüche, wobei die Kanalanordnung (20) zumindest zwei Platten (25) aufweist, die an deren Oberflächen (25a, 25b) aneinander anliegen, wobei an den Oberflächen (25a, 25b) jeweils Nuten (26) ausgebildet sind, welche die Kanalabschnitte (21) definieren.

6. Filtersystem (1) nach einem der voranstehenden Ansprüche, wobei die Versorgungseinrichtung (4) ein erstes Reservoir (41) mit der Absorberflüssigkeit, ein zweites Reservoir (42) mit der Trägerflüssigkeit und einen Tropfengenerator (6) zum Erzeugen des 2-Phasen-Fluidstroms aufweist, wobei der Tropfengenerator (6) mit einem ersten Eingang (61) mit dem ersten Reservoir (41) und mit einem zweiten Eingang (62) mit dem zweiten Reservoir (42) verbunden ist.

7. Filtersystem (1) nach Anspruch 6, wobei der Tropfengenerator (6) einen den ersten Eingang (61) aufweisenden ersten Leitungsabschnitt (61A) und einen den zweiten Eingang (62) aufweisenden zweiten Leitungsabschnitt (62A) aufweist, wobei der erste und der zweite Leitungsabschnitt (61A, 62A) T-förmig ineinander einmünden.

8. Filtersystem (1) nach einem der Ansprüche 1 bis 5, wobei die Versorgungseinrichtung (4) ein Reservoir (41) mit einer Emulsion aus Tropfen der Absorberflüssigkeit und der Trägerflüssigkeit aufweist und das Reservoir (41) mit der Kanalanordnung (20) verbunden ist.

9. Filtersystem (1) nach einem der voranstehenden Ansprüche, wobei die Absorberflüssigkeit Quecksilber oder Galinstan ist, und wobei die Trägerflüssigkeit Öl, insbesondere Silikon-Öl ist.

10. Röntgenapparat (200), mit:
einer Röntgenquelle (210) zum Erzeugen und Abstrahlen von Röntgenstrahlung in einen Strahlengang (215);
einem in dem Strahlengang (215) angeordneten Röntgendetektor (220); und
einem Filtersystem (1) gemäß einem der voranstehenden Ansprüche, wobei die Filtereinrichtung (2) in dem Strahlengang (215) zwischen Röntgenquelle (210) und Röntgendetektor (220) angeordnet ist.

11. Verfahren zur lokalen Veränderung der Intensität von Röntgenstrahlung mit folgenden Schritten:
Erzeugen vorbestimmter Abfolgen von Tropfen aus einem 2-Phasen-Fluidstrom, welcher Tropfen (D) aus einer Röntgenstrahlung absorbierenden Absorberflüssigkeit und eine für Röntgenstrahlung transparente Trägerflüssigkeit (C) enthält; und
Zuführen der Tropfenabfolgen in Kanalabschnitte (21) einer Kanalanordnung (20) einer in einem Strahlengang zwischen einer Röntgenquelle und einem Röntgendetektor angeordneten Filtereinrichtung (2), wobei die Kanalanordnung (20) eine Vielzahl sich in einer Ebene parallel zueinander erstreckender Kanalabschnitte (21) aufweist.

## Claims

1. Filter system (1) for the local attenuation of X-radiation, comprising:
a filter device (2) which is arranged in the beam path (215) of an X-ray apparatus (200) and has a channel arrangement (20) with a multiplicity of channel sections (21) extending parallel to each other on a plane;
a supply device (4) for providing a 2-phase fluid flow containing drops (D) of an absorber liquid which absorbs X-radiation and a carrier liquid that is transparent to X-radiation; and
a sorting section (3) with an input (30) that is connected to the supply device (4), a first output (31) that is connected to the channel arrangement (20), a second output (32), and a deflection device (35) for directing individual drops (D) of the absorber liquid to the first output (31) or the second output (32).

2. Filter system (1) according to claim 1, wherein the channel sections (21) of the channel arrangement (20) are connected to each other via connecting sections (22) in such a way that they form a continuous channel.

3. Filter system (1) according to claim 1, wherein the channel sections (21) of the channel arrangement (20) are each formed by individual channels (23) which are connected to the supply device in each case.

4. Filter system (1) according to one of the preceding claims, wherein a first group of the channel sections (21) of the channel arrangement (20) is arranged on a first plane (E1) and one or more further groups of channel sections (21) are provided, each of these being arranged on planes (E2-E5) which are parallel to the first plane (E1).

5. Filter system (1) according to one of the preceding claims, wherein the channel arrangement (20) has at least two plates (25) which abut each other at their surfaces (25a, 25b), wherein grooves (26) defining the channel sections (21) are formed on the surfaces (25a, 25b) in each case.

6. Filter system (1) according to one of the preceding claims, wherein the supply device (4) has a first reservoir (41) containing the absorber liquid, a second reservoir (42) containing the carrier liquid, and a drop generator (6) for generating the 2-phase fluid flow, wherein the drop generator (6) is connected by means of a first input (61) to the first reservoir (41), and by means of a second input (62) to the second reservoir (42).

7. Filter system (1) according to claim 6, wherein the drop generator (6) has a first line section (61A) comprising the first input (61) and a second line section (62A) comprising the second input (62), wherein the first and the second line sections (61A, 62A) merge with each other in a T-shaped junction.

8. Filter system (1) according to one of claims 1 to 5, wherein the supply device (4) has a reservoir (41) containing an emulsion of drops of the absorber liquid and the carrier liquid, and the reservoir (41) is connected to the channel arrangement (20).

9. Filter system (1) according to one of the preceding claims, wherein the absorber liquid is mercury or Galinstan, and wherein the carrier liquid is oil, in particular silicone oil.

10. X-ray apparatus (200), comprising:
an X-ray source (210) for generating and emitting X-radiation in a beam path (215);
an X-ray detector (220) which is arranged in the beam path (215); and
a filter system (1) according to one of the preceding claims, wherein the filter device (2) is arranged in the beam path (215) between X-ray source (210) and X-ray detector (220).

11. Method for locally changing the intensity of X-radiation, comprising steps as follows:
generating predetermined sequences of drops from a 2-phase fluid flow containing drops (D) of an absorber liquid which absorbs X-radiation and a carrier liquid (C) that is transparent to X-radiation; and
supplying the drop sequences into channel sections (21) of a channel arrangement (20) of a filter device (2) which is arranged in a beam path between an X-ray source and an X-ray detector, wherein the channel arrangement (20) has a multiplicity of channel sections (21) extending parallel to each other on a plane.

## Revendications

1. Système (1) filtrant pour l'affaiblissement local du rayonnement X comprenant :
un dispositif (2) filtrant à monter dans le trajet (215) des rayons d'un appareil (200) à rayons X ayant un agencement (20) de conduit, qui a une pluralité de tronçons (21) de conduit s'étendant parallèlement les uns aux autres dans un plan ;
un dispositif (4) d'alimentation pour disposer d'un courant de fluides à deux phases, qui contient des gouttes (D) composées d'un liquide absorbant le rayonnement X et d'un liquide porteur transparent au rayonnement X ; et
une section (3) de tri, ayant une entrée (30) reliée au dispositif (4) d'alimentation, une première sortie (31) reliée à l'agencement (20) de conduit, une deuxième sortie (32) et un dispositif (35) de déviation pour faire aller des gouttes (D) individuelles du liquide absorbant à la première sortie (31) ou à la deuxième sortie (32).

2. Système (1) filtrant suivant la revendication 1, dans lequel les tronçons (21) de l'agencement (20) de conduit sont reliés entre eux par des tronçons (22) de liaison, de manière à ce que ceux-ci forment un conduit continu.

3. Système (1) filtrant suivant la revendication 1 dans lequel, les tronçons (21) de l'agencement (20) de conduit sont formés chacun de conduits (23) individuels, qui sont reliés respectivement au dispositif d'alimentation.

4. Système (1) filtrant suivant l'une des revendications précédentes, dans lequel un premier groupe des tronçons (21) de l'agencement (20) de conduit est disposé dans un premier plan (E1) et il est prévu un autre groupe ou plusieurs autres groupes de tronçons (21) de conduit, qui sont disposés respectivement dans des plans (E2 à E5) parallèles au premier plan (E1).

5. Système (1) filtrant suivant l'une des revendications précédentes, dans lequel l'agencement (20) de conduit a au moins deux plaques (25), qui s'appliquent l'une à l'autre à leurs surfaces (25a, 25b), dans lequel sur les surfaces (25a, 25b) sont constituées respectivement des rainures (26), qui définissent les tronçons (21) de conduit.

6. Système (1) filtrant suivant l'une des revendications précédentes, dans lequel le dispositif (4) d'alimentation a un premier réservoir (41) ayant le liquide absorbant, un deuxième réservoir (42) ayant le liquide porteur et un générateur (6) de gouttes pour la production du courant de fluide à deux phases, le générateur (6) de gouttes étant relié par une première entrée (61) au premier réservoir (41) et par une deuxième entrée (62) au deuxième réservoir (42).

7. Système (1) filtrant suivant la revendication 6, dans lequel le générateur (6) de gouttes a un premier tronçon (61A) de conduit ayant la première entrée (61) et un deuxième tronçon (62A) de conduit ayant la deuxième entrée (62), le premier et le deuxième tronçon (61A, 62A) de conduit débouchant l'un dans l'autre en forme de T.

8. Système (1) filtrant suivant l'une des revendications 1 à 5, dans lequel le dispositif (4) d'alimentation a un réservoir (41), ayant une émulsion de gouttes du liquide absorbant et du liquide porteur, et le réservoir (41) est relié à l'agencement (20) de conduit.

9. Système (1) filtrant suivant l'une des revendications précédentes, dans lequel le liquide absorbant est du mercure ou du galinstan, et dans lequel le liquide porteur est de l'huile, notamment de l'huile de silicone.

10. Appareil (20) à rayons X, comprenant :
une source (210) de rayons X pour la production et l'émission de rayonnement X suivant un trajet (215) de rayonnement ;
un détecteur (220) de rayons X monté dans le trajet (215) de rayonnement ; et
un système (1) filtrant suivant l'une des revendications précédentes, le dispositif (2) filtrant étant monté dans le trajet (215) de rayonnement, entre la source (210) de rayons X et le détecteur (220) de rayons X.

11. Procédé de modification locale de l'intensité du rayonnement X, comprenant les stades suivants :
production d'une suite déterminée à l'avance de gouttes en un courant de fluide à deux phases, qui contient des gouttes (D) en un liquide absorbant le rayonnement X et en un liquide (C) porteur transparent au rayonnement X et,
envoi de la suite de gouttes dans des tronçons (21) d'un agencement (20) de conduit d'un dispositif (2) filtrant, monté dans un trajet de rayonnement entre une source de rayons X et un détecteur de rayons X, l'agencement (20) de conduit ayant une pluralité de tronçons (21) de conduit s'étendant parallèlement les uns aux autres dans un plan.
